(19) Europäisches Patentamt
European Patent Office
Office européen des brevets

(11) Numéro de publication : **0 167 464**
**B1**

(12) **FASCICULE DE BREVET EUROPÉEN**

(45) Date de publication du fascicule du brevet : **21.10.87**

(51) Int. Cl.⁴ : **C 07 C 69/16**, C 07 C 67/03

(21) Numéro de dépôt : **85420113.4**

(22) Date de dépôt : **21.06.85**

(54) **Procédé de préparation de monocarboxylates d'hydroquinone.**

(30) Priorité : **25.06.84 FR 8410184**

(43) Date de publication de la demande :
**08.01.86 Bulletin 86/02**

(45) Mention de la délivrance du brevet :
**21.10.87 Bulletin 87/43**

(84) Etats contractants désignés :
**AT BE CH DE GB IT LI LU NL SE**

(56) Documents cités :
**EP-A- 0 060 092**
**US-A- 2 588 978**

(73) Titulaire : **RHONE-POULENC CHIMIE**
**25, quai Paul Doumer**
**F-92408 Courbevoie Cedex (FR)**

(72) Inventeur : **Ratton, Serge**
**Hameau de Belmont Vaulx-Milieu**
**F-38090 - Villefontaine (FR)**

(74) Mandataire : **Vignally, Noel et al**
**RHONE-POULENC INTERSERVICES Service Brevets Chimie Centre de Recherches de Saint-Fons B.P. 62**
**F-69192 Saint-Fons Cedex (FR)**

**EP 0 167 464 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé de préparation de monocarboxylates d'hydroquinone à partir de dicarboxylates d'hydroquinone et notamment de monoacétate d'hydroquinone à partir des diacétates d'hydroquinone.

Les monocarboxylates d'hydroquinone se sont révélés comme des produits précieux pour l'obtention des dicarboxylates de monohalogénohydroquinone (en particulier de monochlorohydroquinone) eux-mêmes utilisés pour la préparation de polyesters aromatiques [cf. demande de brevet français n° 79/24 135, publiée sous le n° 2 465 758 et brevet américain n° 4 118 372]. On a constaté en effet que les monocarboxylates d'hydroquinone peuvent être halogénés sélectivement en monocarboxylates de monohalogénohydroquinone, à la différence de l'hydroquinone qui donne naissance à des quantités notables de dihalogénohydroquinone [cf. brevet américain n° 2 743 173] que l'on retrouve sous forme de dicarboxylates de dihalogénohydroquinone après acylation. On a encore constaté que les monocarboxylates d'hydroquinone possèdent l'avantage de pouvoir être halogénés en présence de dicarboxylates d'hydroquinone qui eux demeurent pratiquement inertes lors de l'halogénation. Une telle propriété est favorable à l'halogénation de mélanges de monocarboxylates d'hydroquinone, de dicarboxylates d'hydroquinone et de quantités mineures d'hydroquinone tels que ceux obtenus par acylation partielle de l'hydroquinone.

On ne connaît pas de procédé simple pour accéder aux monocarboxylates d'hydroquinone seuls. On a bien proposé une voie d'accès sélective à ces composés à partir de l'hydroquinone mais sa complexité lui ôte tout intérêt industriel ; en effet ce procédé consiste à protéger un des groupes hydroxyle de l'hydroquinone sous forme d'un groupe fonctionnel duquel l'hydroxyle peut être facilement libéré, puis à acyler l'autre groupe et enfin à régénérer le premier. Une telle méthode a été appliquée par H. S. Olcott, J. Am. Chem. Soc. 59 392 (1937) à la préparation de monoacétate d'hydroquinone par réaction de l'hydroquinone avec le chlorocarbonate de benzyle pour former le carbonate mixte de benzyle et de p-hydroxyphényle lequel est ensuite acétylé en carbonate mixte de benzyle et de p-méthylcarbonyloxyphényle ; dans une troisième étape ce dernier est soumis à une hydrogénolyse en présence de palladium ou de platine. Dès lors le moyen d'accès le plus simple aux monocarboxylates d'hydroquinone réside dans l'acylation partielle de l'hydroquinone qui ne peut être réalisée sans formation simultanée de dicarboxylates d'hydroquinone (cf. H. S. Olcott, loc. cit. et D. Johnston, Chem. Ind. (London) 1982 page 1000). L'emploi de tels mélanges pour la préparation des monocarboxylates de monohalogénohydroquinone se traduit par l'accumulation des dicarboxylates d'hydroquinone. La solution du problème posé par l'obtention sélective de monocarboxylate de monohalogénohydroquinone et des dicarboxylates correspondants passe donc par la mise au point d'un procédé de récupération des dicarboxylates d'hydroquinone et de préparation des monocarboxylates d'hydroquinone. La présente invention a précisément ce double objet.

Plus spécifiquement la présente invention a pour objet un procédé de préparation de monocarboxylates d'hydroquinone à partir de dicarboxylates d'hydroquinone, caractérisé en ce que l'on fait réagir un excès d'un dicarboxylate d'hydroquinone avec de l'hydroquinone éventuellement en présence d'un catalyseur.

La réaction des dicarboxylates d'hydroquinone avec l'hydroquinone, qui sera appelée par la suite « réaction de redistribution » ou « redistribution », peut être représentée par le schéma réactionnel suivant :

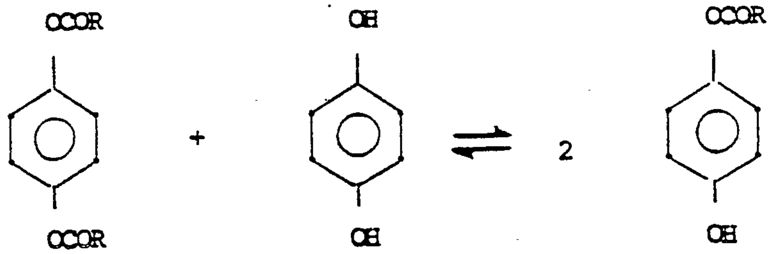

(1)

C'est une réaction équilibrée qui aboutit à la formation d'un mélange de monocarboxylate d'hydroquinone, de dicarboxylate d'hydroquinone et d'hydroquinone. Sa composition dépend des conditions de la réaction. Ce mélange peut être utilisé pour la préparation sélective de monocarboxylates de monohalogénohydroquinone par halogénation du monocarboxylate d'hydroquinone qu'il contient. Dans ce cas il importe qu'il reste le moins possible d'hydroquinone dans le mélange puisque lors de l'halogénation ultérieure ce composé donne naissance à des dérivés dihalogénés indésirables. C'est la raison pour laquelle on opère en présence d'un excès de dicarboxylate d'hydroquinone qui permet de déplacer l'équilibre de la réaction de façon à favoriser la formation du monocarboxylate d'hydroquinone.

En règle générale l'excès de dicarboxylate d'hydroquinone est d'au moins 0,5 mole par rapport à la quantité stœchiométrique résultant du schéma réactionnel (1). Il n'y a pas de valeur supérieure critique de

l'excès de dicarboxylate d'hydroquinone, toutefois, au-delà d'une certaine valeur, le gain réalisé sur la transformation de l'hydroquinone est contrebalancé par l'inconvénient d'avoir à recycler des quantités importantes de dicarboxylate d'hydroquinone. Dès lors il n'est pas nécessaire de mettre en œuvre un excès de dicarboxylate d'hydroquinone supérieur à 4 moles par rapport à la quantité stœchiométrique. En résumé la quantité de dicarboxylates d'hydroquinone, exprimée en mole par mole d'hydroquinone est d'au moins 1,5 et de préférence comprise dans un intervalle de 1,5 à 5.

La réaction de redistribution peut être conduite en masse ou dans un solvant à la température de réaction des dicarboxylates d'hydroquinone, de l'hydroquinone et des monocarboxylates d'hydroquinone, inerte dans les conditions de la réaction. Dans ce dernier cas on utilise plus particulièrement :

— des acides carboxyliques liquides dans les conditions de la réaction et de préférence à température ambiante on peut citer notamment des acides alcanoïques comportant de 1 à 8 atomes de carbone tels que les acides formique, acétique, propionique, butyrique, isobutyrique, méthyl-2 butanoïque, éthyl-2 butanoïque, diméthyl-2,2 butanoïque, pentanoïque, méthyl-2 pentanoïque, méthyl-5 pentanoïque, hexanoïque éthyl-2 hexanoïque. De préférence on utilise l'acide carboxylique dont dérive le dicarboxylate d'hydroquinone,

— des éthers aliphatiques ou hétérocycliques tels que : l'oxyde d'éthyle, de n-propyle, d'isopropyle, le tétrahydrofuranne, le dioxane,

— des hydrocarbures aliphatiques saturés, cycloaliphatiques saturés ou aromatiques tels que le n-hexane, le cyclohexane, le toluène, le benzène,

— des halogénoalcanes tels que le chloroforme, le tétrachlorure de carbone, le trichloroéthylène,

— des hydrocarbures halogénoaromatiques tels que le chlorobenzène.

La température de la réaction de redistribution peut varier dans de larges limites. En général des températures comprises dans un intervalle de 50 à 250 °C et de préférence de 80 à 180 °C conviennent bien. La réaction peut être conduite à pression normale ou sous pression ; lorsque la température choisie est supérieure au point d'ébullition de certains des constituants du mélange on peut opérer sous la pression autogène des réactifs.

Lorsqu'on conduit la redistribution dans un acide carboxylique on peut opérer en présence ou en absence de catalyseur. Lorsqu'on utilise un catalyseur on fait appel à des acides minéraux ou organiques forts, c'est-à-dire des acides présentant un pK dans l'eau à 25 °C inférieur à 1. On fait appel de préférence à l'acide sulfurique et aux acides sulfoniques tels que les acides méthanesulfonique, di- et trifluorométhanesulfonique, benzènesulfonique, toluènesulfonique, naphtalènesulfonique ou aux résines sulfoniques.

La quantité d'acide fort exprimée en équivalent de protons par mole d'hydroquinone peut varier dans de larges limites. En général elle est comprise entre 0,000 1 et 0,2 équivalent de proton par mole d'hydroquinone.

Lorsqu'on utilise un solvant autre qu'un acide et en particulier un éther, on peut opérer en présence d'un catalyseur du type de ceux qui sont utilisés dans les réactions de transestérification. A cet effet on peut faire appel à des bases azotées organiques telles que les amines primaires, secondaires ou tertiaires et les bases hétérocycliques ; on peut citer plus particulièrement la diéthylamine, l'éthylamine, la triéthylamine, la n-propylamine, la diisopropylamine, la triéthanolamine, la pyridine, la pipéridine. On peut également faire appel à des carboxylates alcalins tels que les acétates de K, Li ou Na et à des acides de Lewis tels que ceux cités dans l'ouvrage de G. A. Olah, Friedel-Crafts Reaction volume 1, pages 191 à 291. On fait appel de préférence à des sels de zinc, de titane, de manganèse, de cobalt ou à des alkoxydes métalliques. On peut citer notamment des halogénures de zinc tels que $ZnCl_2$ ; des titanates d'alkyle tels que les titanates de méthyle, d'éthyle, de n-propyle, d'isopropyle, de n-butyle, d'isobutyle ; des carboxylates de Mn et de Co tels que les acétate, propionate et isobutyrate de Mn et de Co.

La quantité de catalyseur exprimée en mole par mole d'hydroquinone peut être comprise dans un intervalle de 0,000 1 à 0,2 mole par mole d'hydroquinone.

En fin de réaction les constituants du mélange peuvent être séparés par distillation et le dicarboxylate d'hydroquinone et l'hydroquinone recyclés à la préparation du monocarboxylate d'hydroquinone. Lorsqu'on désire préparer des monocarboxylates de monohalogénohydroquinone, il est préférable de procéder à l'halogénation directement sur le mélange de redistribution, le cas échéant après avoir éliminé le solvant et le catalyseur de redistribution. Dans le cas où on a fait appel à un acide alcanoïque comme solvant, l'halogénation peut avoir lieu directement sur le mélange de redistribution.

Les dicarboxylates d'hydroquinone mis en œuvre peuvent être obtenus par acylation complète de l'hydroquinone au moyen des agents acylants usuels tels que les anhydrides et chlorure d'acides.

Le procédé de l'invention convient tout spécialement bien pour la préparation de monocarboxylates d'hydroquinone de formule générale :

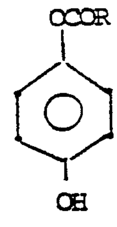

(I)

dans laquelle R représente un radical alkyle linéaire ou ramifié ayant de 1 à 4 atomes de carbone, à partir des dicarboxylates d'hydroquinone correspondant.

Comme exemples de radicaux R on peut citer les radicaux méthyle, éthyle, n-propyle, isopropyle, n-butyle, isobutyle.

Les exemples suivants illustrent l'invention et montrent comment elle peut être mise en pratique.

Exemple 1

Dans un réacteur en verre de 500 ml, équipé d'un agitateur et résistant à la pression on charge :

| | | |
|---|---|---|
| - hydroquinone | 26,4 g | (0,24 mole) |
| - diacétate d'hydroquinone | 139,68 g | (0,72 mole) |
| - éther diisopropylique | 200 ml | |
| - triéthylamine | 1,2 g | (0,012 mole) |

On porte la température à 150 °C pendant 3 heures à pression autogène. On chasse ensuite le solvant et l'amine. On obtient un résidu solide beige clair dont le poids est de 168,9 g. Par analyse en chromatographie phase liquide, on a identifié et dosé les produits suivants :

| | % pondéral dans le produit brut | Moles | Réactifs transformés * ou formés ** |
|---|---|---|---|
| diacétate d'hydroquinone | 56,2 | 0,489 | 0,231 * |
| monoacétate d'hydroquinone | 39,2 | 0,435 | 0,435 ** |
| hydroquinone | 1,7 | 0,026 | 0,214 * |

Taux de transformation du diacétate d'hydroquinone : 32,1 %.

Taux de transformation de l'hydroquinone : 89,2 %.

Rendement en monoacétate d'hydroquinone par rapport à l'hydroquinone et à son diester consommés : 100 %.

Exemple 2

Dans un réacteur en verre de 200 ml, équipé d'un système d'agitation, et d'un thermomètre, d'un réfrigérant ascendant et d'un dispositif de chauffage on charge :

| | | |
|---|---|---|
| - hydroquinone | 9,8 g | (0,0891 mole) |
| - diacétate d'hydroquinone | 51,8 g | (0,267 mole) |
| - acide acétique | 246 ml | |
| - acide p-toluènesulfonique | 0,64 g | |

On porte le mélange homogène à reflux pendant 3 heures. On élimine ensuite l'acide acétique par distillation sous pression atmosphérique. On obtient après refroidissement un résidu solide blanc dont le poids est de 32,8 g.

Par analyse en chromatographie phase liquide, on identifie et dose les produits suivants :

| | | |
|---|---|---|
| diacétate d'hydroquinone | 32,8 g | (0,169 mole) |
| monoacétate d'hydroquinone | 27,06 g | (0,178 mole) |
| hydroquinone | 0,96 g | (0,0087 mole). |

Le rendement en monoacétate d'hydroquinone est quantitatif par rapport aux réactifs diacétate d'hydroquinone et hydroquinone transformés.

Exemple 3

Dans un réacteur en verre de 500 ml, équipé comme à l'exemple 2 on charge :

| | | |
|---|---|---|
| - hydroquinone | 8,92 g | (0,081 mole) |
| - diacétate d'hydroquinone | 47,18 g | (0,2432 mole) |
| - acide acétique | 224 ml | |
| - acide p-toluènesulfonique | 0,58 g | |

On porte le mélange à ébullition pendant 3 heures. On dose les produits en solution dans la solution acétique par chromatographie en phase liquide.

| Composés | Chargés mmoles | Dosés après réaction mmoles | transformés ou formés mmoles |
|---|---|---|---|
| diacétate d'hydroquinone | 243,2 | 163,6 | 79,6 |
| monoacétate d'hydroquinone | | 152,5 | 152,5 |
| hydroquinone | 81 | 7,9 | 73,1 |

Taux de transformation de l'hydroquinone : 90,2 %.
Taux de transformation du diacétate d'hydroquinone : 32,7 %.
Rendement en monoacétate d'hydroquinone par rapport à l'hydroquinone et au diacétate d'hydroquinone transformés : 100 %.

Exemple 4

Dans un réacteur de 200 ml, équipé d'un agitateur, et résistant à la pression on charge :

| | | |
|---|---|---|
| - hydroquinone | 11 g | (0,1 mole) |
| - diacétate d'hydroquinone | 58,2 g | (0,3 mole) |
| - acide acétique | 100 ml | |
| - acide p.toluène sulfonique | 0,5 g. | |

On porte le mélange à la température de 200 °C sous pression autogène que l'on maintient pendant 2 heures.

On refroidit la masse réactionnelle et on la dilue avec de l'éther diisopropylique. Un dosage par chromatographie liquide sous haute pression de la solution éthérée donne les résultats suivants :

| | | |
|---|---|---|
| hydroquinone | 1,31 g | (0,012 mole) |
| diacétate d'hydroquinone | 39,3 g | (0,203 mole) |
| monoacétate d'hydroquinone | 28 g | (0,184 mole) |

Taux de transformation de l'hydroquinone : 88 %
Taux de transformation du diacétate d'hydroquinone : 32 %
Rendement en monoacétate d'hydroquinone par rapport au diacétate d'hydroquinone et à l'hydroquinone transformés : 100 %.

Exemple 5

On opère comme dans l'exemple 1 mais en remplaçant l'éther diisopropylique par le tétrachlorure de carbone. La réaction a été effectuée à 150 °C pendant une nuit.

Le taux de transformation de l'hydroquinone est de 89 %.

Le rendement est quantitatif en monoester par rapport au diester et à l'hydroquinone transformés.

Exemple 6

On opère comme dans l'exemple 1 mais en supprimant le solvant : on opère en milieu fondu à 150 °C sans catalyseur.

Charges :

| | | |
|---|---|---|
| hydroquinone | 53 g | (0,482 mole) |
| diacétate d'hydroquinone | 291 g | (1,5 mole). |

Composition de la masse réactionnelle en fin de réaction (analyse par chromatographie liquide sous haute pression) :

| | | |
|---|---|---|
| hydroquinone | 4,78 g | (0,043 mole) |
| diacétate d'hydroquinone | 207 g | (1,07 mole) |
| monoacétate d'hydroquinone | 140 g | (0,02 mole) |

**Revendications**

1. Procédé de préparation de monocarboxylates d'hydroquinone à partir de dicarboxylates d'hydroquinone, caractérisé en ce que l'on fait réagir un excès d'un dicarboxylate d'hydroquinone avec de l'hydroquinone éventuellement en présence d'un catalyseur, à une température comprise entre 50 et 250 °C.

2. Procédé selon la revendication 1 caractérisé en ce que l'on opère dans un solvant inerte.

3. Procédé selon la revendication 2 caractérisé en ce que l'on utilise comme solvant un acide alcanoïque, un éther, un hydrocarbure aliphatique saturé, cycloaliphatique saturé, aromatique ou leurs dérivés halogénés.

4. Procédé selon l'une quelconque des revendications 1 à 3 caractérisé en ce que l'on opère en présence d'un catalyseur de transestérification.

5. Procédé selon la revendication 4 caractérisé en ce que l'on utilise comme catalyseur un acide fort.

6. Procédé selon l'une quelconque des revendications 1 à 5 caractérisé en ce que la quantité de dicarboxylate exprimée en mole par mole d'hydroquinone est d'au moins 1,5.

7. Procédé selon l'une quelconque des revendications 4, 5 et 6 caractérisé en ce que la quantité de catalyseur acide exprimée en équivalent de proton par mole d'hydroquinone est comprise entre 0,000 1 et 0,2.

8. Procédé selon les revendications 4 et 6 caractérisé en ce que l'on utilise un catalyseur pris dans le groupe des carboxylates alcalins, des bases azotées et des acides de Lewis.

9. Procédé selon la revendication 8 caractérisé en ce que la quantité de catalyseur exprimée en mole par mole d'hydroquinone est comprise dans un intervalle de 0,000 1 à 0,2.

10. Procédé selon l'une quelconque des revendications 1 à 9 caractérisé en ce que la température de la réaction est comprise dans un intervalle de 80 à 180 °C.

11. Procédé selon l'une quelconque des revendications 1 à 10, caractérisé en ce que le dicarboxylate d'hydroquinone est le diacétate et que l'on prépare le monoacétate d'hydroquinone.

**Claims**

1. Process for preparing hydroquinone monocarboxylates from hydroquinone dicarboxylates, characterized in that an excess of hydroquinone dicarboxylate is reacted with hydroquinone, in the presence of a catalyst if desired, at a temperature of between 50 and 250 °C.

2. Process according to Claim 1, characterized in that it is carried out in an inert solvent.

3. Process according to Claim 2, characterized in that the solvent employed is an alkanoic acid, an ether, a saturated aliphatic, saturated cycloaliphatic or aromatic hydrocarbon or halogenated derivatives thereof.

4. Process according to any one of Claims 1 to 3, characterized in that it is carried out in the presence of a transesterification catalyst.

5. Process according to Claim 4, characterized in that a strong acid is employed as a catalyst.

6. Process according to any one of Claims 1 to 5, characterized in that the quantity of dicarboxylate expressed in mole per mole of hydroquinone is at least 1.5.

7. Process according to any one of Claims 4, 5 and 6, characterized in that the quantity of acidic catalyst expressed in proton equivalent per mole of hydroquinone is between 0.000 1 and 0.2.

8. Process according to Claims 4 and 6, characterized in that a catalyst taken from the group of alkali metal carboxylates, nitrogenous bases and Lewis acids is employed.

9. Process according to Claim 8, characterized in that quantity of catalyst expressed in mole per mole of hydroquinone is in a range from 0.000 1 to 0.2.

10. Process according to any one of Claims 1 to 9, characterized in that the reaction temperature is in a range from 80 to 180 °C.

11. Process according to any one of Claims 1 to 10, characterized in that the hydroquinone dicarboxylate is the diacetate and that hydroquinone monoacetate is prepared.

**Patentansprüche**

1. Verfahren zur Herstellung von Hydrochinon-monocarboxylaten, ausgehend von Hydrochinondicarboxylaten, dadurch gekennzeichnet, daß man einen Überschuß eines Hydrochinon-dicarboxylats mit Hydrochinon, gegebenenfalls in Gegenwart eines Katalysators, bei einer Temperatur zwischen 50 und 250 °C zur Reaktion bringt.

2. Verfahren gemäß Anspruch 1, dadurch gekennzeichnet, daß man in einem inerten Lösungsmittel arbeitet.

3. Verfahren gemäß Anspruch 2, dadurch gekennzeichnet, daß man als Lösungsmittel eine Alkansäure, einen Ether, einen gesättigten aliphatischen, gesättigten cycloaliphatischen, aromatischen Kohlenwasserstoff oder deren halogenierte Derivate verwendet.

4. Verfahren gemäß einem der Ansprüche 1 bis 3, dadurch gekennzeichnet, daß man in Gegenwart eines Umesterungskatalysators arbeitet.

5. Verfahren gemäß Anspruch 4, dadurch gekennzeichnet, daß man als Katalysators eine starke Säure verwendet.

6. Verfahren gemäß einem der Ansprüche 1 bis 5, dadurch gekennzeichnet, daß die Menge an Dicarboxylat, ausgedrückt in Mol pro Mol Hydrochinon, mindestens 1,5 ist.

7. Verfahren gemäß einem der Ansprüche 4, 5 und 6 dadurch gekennzeichnet, daß die Menge an saurem Katalysator, ausgedrückt in protonenäquivalent/Mol Hydrochinon, zwischen 0,000 1 und 0,2 beträgt.

8. Verfahren gemäß Anspruch 4 und 6, dadurch gekennzeichnet, daß man einen Katalysator, ausgewählt aus der Gruppe der Alkalicarboxylate, Stickstoffbasen und Lewissäuren, verwendet.

9. Verfahren gemäß Anspruch 8, dadurch gekennzeichnet, daß die Katalysatormenge, ausgedrückt in Mol pro Mol Hydrochinon, in dem Bereich zwischen 0,000 1 und 0,2 liegt.

10. Verfahren gemäß einem der Ansprüche 1 bis 9, dadurch gekennzeichnet, daß die Temperatur der Reaktion in einem Intervall von 80 bis 180 °C liegt.

11. Verfahren gemäß einem der Ansprüche 1 bis 10, dadurch gekennzeichnet, daß das Hydrochinondicarboxylat das Diacetat ist und daß man das Hydrochinon-monoacetat herstellt.